# EUROPEAN PATENT SPECIFICATION

(11) **EP 0 506 772 B1**
(45) Date of publication and mention of the grant of the patent: **23.06.1999**
(21) Application number: 91901491.0
(22) Date of filing: 18.12.1990
(51) Int. Cl.: A61K 31/70

(54) **A PHARMACEUTICAL COMPOSITION COMPRISING A PHOSPHOSUGAR**
PHOSPHOZUCKER ENTHALTENDE PHARMAZEUTISCHE ZUBEREITUNG
COMPOSITION PHARMACEUTIQUE COMPRENANT UN PHOSPHOSUCRE

(30) Priority: 21.12.1989 SE 8904354
(43) Date of publication of application: 07.10.1992
(73) Proprietor: PERSTORP AB, 284 80 Perstorp (SE)
(72) Inventor: Sirén, Matti, CH-6926 Montagnola (CH)
(74) Representative: Hansen, Bernd, Dr. Dipl.-Chem.
(86) International application number: SE9000843
(87) International publication number: WO9109604

(56) References cited:
- DE-A- 2 508 474
- DE-A- 2 629 845
- DE-A- 3 109 202
- FR-A- 2 529 459
- FR-M- 0 006 065
- FR-M- 0 007 346
- GB-A- 1 158 456

## Description

The present invention relates to a pharmaceutical composition comprising as a pharmaceutically active ingredient a triphosphorylated sugar compound or a salt thereof, which sugar compound is or is derived from a pentose, a hexose or a heptose.

The invention also relates to the use of said compound for the preparing of a medicament for preventing, alleviating or combatting different conditions in mammals including man.

There exist many diseases and conditions, which are not treated in a correct way. The underlying components resulting in different diseases are badly understood and in many cases only the symptoms of a disease can be treated with existing drugs without any reversal of the disease per se.

This phenomenon is valid for conditions, such as tissue damage, diabetic complications, cardiovascular diseases, etc.

According to the present invention it has quite unexpectedly been found possible to treat conditions and diseases belonging to the above described group.

Thus, a pharmaceutical composition comprising as a pharmaceutically active ingredient a phosphorylated sugar compound according to claim 1 a salt thereof has been brought about. The composition also comprises a pharmaceutically acceptable carrier, excipient or additive therefore.

When said phosphorylated sugar compound is in salt form the salts of sodium, potassium, calcium, zinc or magnesium are preferred. Other salts could also comprise the compound and a mixture of different salts or a combination of the compound in acid and salt form is also within the scope of the invention.

It is suitable that the composition according to the invention exists in a unit dosage form. Tablets, granules and capsules are suitable administration forms for such a unit dosage. The tablets can furthermore be treated in different ways to protect the compound from uncontrolled hydrolysis in the stomach and intestine and to provide a desired absorption. Other suitable administration forms are e.g. slow release formulation, transdermal formulations, nasal, rectal, intra-articular, intraperitoneal and subcutaneous administration.

In some cases, it is preferable to use the unit dosage in the form of a solution for intravenous administration.

The unit dosage form comprises 0.01g to 2.0g of the compound according to the invention per unit dosage. Preferably the content of the compound is 0.05g to 2.0g and most preferably 0.10g to 2.0g.

The pharmaceutical composition can in addition also comprise another pharmaceutically active ingredient. In these cases the amount of the compound according to the invention is 5% to 95% and preferably 10% to 95% by weight of the active ingredients.

Said compounds are phosphosugars having a degree of substitution of three concerning the phosphate groups.

In one embodiment of the invention the compound is a phosphorylated aldopentose with a degree of substitution of three.

In another embodiment of the invention the compound is a phosphorylated ketopentose with a degree of substitution of three.

In still another embodiment of the invention the compound is a phosphorylated aldohexose with a degree of substitution of three.

In even another embodiment of the invention the compound is a phosphorylated ketohexose with a degree of substitution of three.

In yet another embodiment of the invention the compound is a phosphorylated heptose with a degree of substitution of three.

Preferably the compound is in a cyclic form either in α- or β-form.

Specific embodiments of the invention comprises a pharmaceutical composition, wherein the pentose, hexose or heptose is selected front the group consisting of D/L-ribose, D/L-arabinose, D/L-xylose, D/L-lyxose, D/L-allose, D-altrose, D/L-glucose, D/L-mannose, D/L-gulose, D-idose, D/L-galactose, D/L-talose, D/L-glucoheptose, D/L-mannoheptose, D/L-ribulose, D/L-xylulose, D/L-psicose, D/L-fructose, D/L-sorbose and D/L-tagatose.

The compounds of the present invention can be produced in per se known ways i.e. phosphorylation of the corresponding non-phosphorylated substances and then separated and purified with techniques using for example ionic forces as a principle.

In order to get a sufficient phosphorylation preferably a mixture of ortophosphoric acid and polyphosphoric acid is used. Also other phosphorylating agents can be contemplated such as phosphorochloridates, phosphoramidites and alkoxyalkylaminophosphines

After phosphorylation the obtained species are separated with e.g. ion exchange chromatographic techniques. Elution of an ion exchange column with a proper eluent such as hydrochloric acid with increasing concentration results in different fractions containing phosphorylated species with different degrees of substitution and different isomeric structures. Precipitation with different cations is required in order to get pure salts of the different compounds of the present invention.

The present invention also relates to the use of the present phosphorylated sugar compound for the preparing of a medicament for preventing, alleviating or combatting the following conditions: Tissue damage such as oedema formation, vascular leakage, burns, rhinitis and asthma; bone disorders such as osteoporosis, Paget's disease, bone erosion and hypercalcemia; diabetes or complications thereof such as cataract formation, retinopathy, vascular complications, neuropathy, nephropathy, hyperglycemia and hyperketonemia; disorders related to transplantation and graft operations such as rejection; abnormal immunological reactions; abnormal levels of lipoproteins; cardiovascular diseases such as atherosclerosis; hypertension; thrombosis; hemorrhage; conditions of ischemia and reperfusion; conditions of shock; organ injury and damage to cells such as endothelial cells, platelets, and erythrocytes.

The invention also covers the use of the present compounds for the preparing of a medicament for preventing, alleviating or combatting conditions related to vasculitis, dermatitis, gastrointestinal diseases such as ulcerative colitis and pancreatitis, synovitis, periodontal diseases, cerebral diseases, eye diseases or damage to the retina or lens, light and oxygen induced diseases or damages, autoimmune diseases such as multiple sclerosis and metal intoxication.

The medicament is in preferred embodiments in the form of tablets, granules or solutions. For administration to human patients appropriate dosages can routinely be determined by those skilled in the art by extension of results obtained in animals at various dosages. The preferred dosage falls within the range by 0.1 mg to 1000 mg, especially 0.1 mg to 200, mg/day/kg body weight of the compound according to the invention.

The medicament according to the present invention contains a sugar compound preferably selected from those as claimed in claim 2.

In one preferred embodiment of the invention the medicament is prepared from two or more compounds of the present invention. Physical mixing of two or more compounds or two or more compounds with covalent bondings then are constituents of the medicament. When the compounds are covalently bonded preferably dimeric, trimeric or tetrameric forms of similar or different phosphosugars are used.

The function of the medicament is to reverse, prevent or alleviate specific types of cell to cell attachement which are deleterious to the body.

For example granulocytes, neutrophils, etc., i.e. cells with diverse functions, are activated under some physiological or pathological conditions and adhere to other cell types such as endothelial cells. The adhesion process leads to different forms of cytotoxicity, phagocytosis, chemotaxis and induction of cell proliferation and differentiation. These events often leads to conditions described above such as tissue damage, cardiovascular diseases and other types or diseases.

The cell to cell adhesion process is regulated by specific types of receptors. The mode of action of the medicament is to regulate the receptor function with primarily an antagonistic effect.

Furthermore the above-mentioned process often involves cell damage and cell destruction caused by certain enzymes such as hydrolases, proteases and the like. Many of these enzymes for example lysosomal enzymes are activated via processes involving receptor interactions.

The medicament also interacts with these types of receptors with a beneficial regulation as a consequence.

The medicament acts via cytoprotection and stabilization of e.g. cell membranes.

For a proper receptor interactions the stereochemical environment around C₂ and C₃ as well as C₅ and C₆ respectively are essential; Cₙ in this context relates to the number the different carbon atoms according to the valid nomenclature.

## Claims (Claims for the following Contracting State(s): AT, BE, CH, DE, DK, FR, GB, IT, LI, LU, NL, SE)

1. A pharmaceutical composition comprising at least one pharmaceutically active ingredient being selected from a phosphorylated aldopentose, ketopentose, aldohexose, ketohexose or a heptose, all phosphorylated sugars having a degree of substitution of three.

2. A pharmaceutical composition according to Claim 1, wherein the pentose hexose or heptose is selected from D/L-ribose, D/L-arabinose, D/L-xylose, D/L-lyxose, D/L-allose, D-altrose, D/L-glucose, D/L-mannose, D/L-gulose, D-idose, D/L-galactose, D/L-talose, D/L-glucoheptose, D/L-mannoheptose, D/L-ribulose, D/L-xylulose, D/L-psicose, D/L-fructose, D/L-sorbose or D/L-tagatose.

## Claims (Claims for the following Contracting State(s): ES, GR)

1. A process for preparing a pharmaceutical composition comprising formulating as a pharmaceutically active ingredient a phosphorylated aldopentose, ketopentose, aldohexose, ketohexose or a heptose, all phosphorylated sugars having a degree of substitution of three with a pharmaceutically acceptable carrier, excipient or additive thereof.

2. The process according to claim 1 wherein the pentose, hexose or heptose is selected from D/L-ribose, D/L-arabinose, D/L-xylose, D/L-lyxose, D/L-allose, D-altrose, D/L-glucose, D/L-mannose, D/L-gulose, D-idose, D/L-galactose, D/L-talose, D/L-glucoheptose, D/L-mannoheptose, D/L-ribulose, D/L-xylulose, D/L-psicose, D/L-fructose, D/L-sorbose or D/L-tagatose.

## Patentansprüche (Patentansprüche für folgende(n) Vertragsstaat(en): AT, BE, CH, DE, DK, FR, GB, IT, LI, LU, NL, SE)

1. Pharmazeutische Zusammensetzung, umfassend wenigstens einen pharmazeutischen Wirkstoff, ausgewählt aus einer phosphorylierten Aldopentose, Ketopentose, Aldohexose, Ketohexose oder Heptose, wobei alle phosphorylierten Zucker einen Substitutionsgrad von drei besitzen.

2. Pharmazeutische Zusammensetzung nach Anspruch 1, wobei die Pentose, Hexose oder Heptose ausgewählt ist aus D/L-Ribose, D/L-Arabinose, D/L-Xylose, D/L-Lyxose, D/L-Allose, D-Altrose, D/L-Glukose, D/L-Mannose, D/L-Gulose, D-Idose, D/L-Galaktose, D/L-Talose, D/L-Glucoheptose, D/L-Mannoheptose, D/L-Ribulose, D/L-Xylulose, D/L-Psicose, D/L-Fructose, D/L-Sorbose oder D/L-Tagatose.

## Patentansprüche (Patentansprüche für folgende(n) Vertragsstaat(en): ES, GR)

1. Verfahren zur Herstellung einer pharmazeutischen Zusammensetzung, umfassend das Formulieren einer phosphorylierten Aldopentose, Ketopentose, Aldohexose, Ketohexose oder Heptose als pharmazeutischem Wirkstoff, wobei alle phosphorylierten Zucker einen Substitutionsgrad von drei besitzen, mit einem pharmazeutisch verträglichen Träger, Exzipienten oder Zusatzstoff.

2. Verfahren nach Anspruch 1, wobei die Pentose, Hexose oder Heptose ausgewählt ist aus D/L-Ribose, D/L-Arabinose, D/L-Xylose, D/L-Lyxose, D/L-Allose, D-Altrose, D/L-Glukose, D/L-Mannose, D/L-Gulose, D-Idose, D/L-Galaktose, D/L-Talose, D/L-Glucoheptose, D/L-Mannoheptose, D/L-Ribulose, D/L-Xylulose, D/L-Psicose, D/L-Fructose, D/L-Sorbose oder D/L-Tagatose.

## Revendications (Revendications pour l'(les) Etat(s) contractant(s) suivant(s): AT, BE, CH, DE, DK, FR, GB, IT, LI, LU, NL, SE)

1. Composition pharmaceutique comprenant au moins un constituant pharmaceutiquement actif choisi parmi un aldopentose, un cétopentose, un aldohexose, un cétohexose ou un heptose phosphorylé, tous les sucres phosphorylés ayant un degré de substitution de trois.

2. Composition pharmaceutique selon la revendication 1, dans laquelle le pentose, l'hexose ou l'heptose est choisi parmi le D/L-ribose, le D/L-arabinose, le D/L-xylose, le D/L-lyxose, le D/L-allose, le D-altrose, le D/L-glucose, le D/L-mannose, le D/L-gulose, le D-idose, le D/L-galactose, le D/L-talose, le D/L-glucoheptose, le D/L-mannoheptose, le D/L-ribulose, le D/L-xylulose, le D/L-psicose, le D/L-fructose, le D/L-sorbose ou le D/L-tagatose.

## Revendications (Revendications pour l'(les) Etat(s) contractant(s) suivant(s): ES, GR)

1. Procédé de préparation d'une composition pharmaceutique comprenant la formulation comme constituant pharmaceutiquement actif d'un aldopentose, d'un cétopentose, d'un aldohexose, d'un cétohexose ou d'un heptose phosphorylé, tous les sucres phosphorylés ayant un degré de substitution de trois, avec un véhicule, un excipient ou un additif à ceux-ci pharmaceutiquement acceptable.

2. Procédé selon la revendication 1, dans lequel le pentose, l'hexose ou l'heptose est choisi parmi le D/L-ribose, le D/L-arabinose, le D/L-xylose, le D/L-lyxose, le D/L-allose, le D-altrose, le D/L-glucose, le D/L-mannose, le D/L-gulose, le D-idose, le D/L-galactose, le D/L-talose, le D/L-glucoheptose, le D/L-mannoheptose, le D/L-ribulose, le D/L-xylulose, le D/L-psicose, le D/L-fructose, le D/L-sorbose ou le D/L-tagatose.
